(19) Europäisches Patentamt

European Patent Office

Office européen des brevets

(11) **EP 4 368 024 A1**

(12) **EUROPEAN PATENT APPLICATION**

(43) Date of publication:
**15.05.2024 Bulletin 2024/20**

(21) Application number: **22207309.0**

(22) Date of filing: **14.11.2022**

(51) International Patent Classification (IPC):
*A01N 59/00* (2006.01)    *A01N 41/06* (2006.01)
*A01N 25/12* (2006.01)    *A01N 25/24* (2006.01)
*A01N 25/30* (2006.01)    *A01P 1/00* (2006.01)

(52) Cooperative Patent Classification (CPC):
(C-Sets available)
**A01P 1/00; A01N 41/06; A01N 59/00**        (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Klareco S.r.l.**
**26020 Palazzo Pignano (CR) (IT)**

(72) Inventor: **OLMO, Federico**
**Palazzo Pignano (CR) (IT)**

(74) Representative: **Villa, Livia**
**ADV IP S.r.l.**
**Corso di Porta Vittoria 29**
**20122 Milano (IT)**

(54) **IMPROVED POWDER COMPOSITION FOR DISINFECTION OF THE TEATS OF DAIRY ANIMALS**

(57)    An improved disinfectant composition in powder form for the post-milking teat disinfection of dairy animals is described. The composition has been proved to be well tolerable from the dermatological point of view, has a very appreciable persistence on skin, as well as a long-term storage stability.

(52) Cooperative Patent Classification (CPC): (Cont.)

C-Sets
**A01N 41/06, A01N 25/12, A01N 25/24,
A01N 25/32;
A01N 59/00, A01N 25/12, A01N 25/24,
A01N 25/30, A01N 41/06**

**Description**

FIELD OF THE INVENTION

The present invention relates to an improved disinfectant composition in powder form, suitable in particular for the post-milking teat disinfection of dairy animals. The composition has been proved to be well tolerable from the dermatological point of view, has a very appreciable persistence on skin, as well as a long-term storage stability.

BACKGROUND OF THE INVENTION

Products having a disinfectant action to protect the mammary system in dairy animals from contamination by microorganisms such as bacteria and fungi that are the cause of recurring infections are known.

The disinfectants currently used for this purpose are in fluid state and are in the form of more or less viscous liquids.

These disinfectants, as being marketed in liquid form and packaged in large containers, require large spaces for storage before use.

Furthermore, organic chlorine-based products are known, also used in preparations intended for human use, such as skin disinfectants. In the veterinary field, the use of known organic chlorine-based compositions for the post-milking teat disinfection of dairy animals presents several drawbacks that can be primarily ascribable to the high dosages that must be used to obtain significant advantageous effects.

A first drawback attributable to the use of organic chlorine compounds at high dosage is associated, for example, to the irritant and inflammatory effect to the skin of the animals; in addition, if inhaled, it is an irritant to the respiratory tracts thereof. The onset of said side effects may require that treatment be discontinued before the desired results have been achieved. Moreover, a high dosage of chlorine compounds can result in irritant and inflammatory effects to the operator during use of the product.

EP2730168 discloses a powder composition suitable for disinfecting dairy animals' teats post-milking, comprising chloramine T as active agent, together with sorbitol. The presence of high amounts of sorbitol together with the active agent achieves the desired disinfectant activity, ensuring at the same time a prolonged emollient effect over time. Moreover, the presence of sorbitol and a natural gum allows to prolong the contact time of the composition with the areas to be disinfected, making it possible to achieve the desired effect with significantly lower quantities of chlorine compounds compared to the concentrations known in the art.

The composition of EP2730168 however presents some aspects that would be desirable to improve, such as the stability and flowability of the powder, and the homogeneity of the product after dissolution of the powder in water. The current limitations are in particular caused by the presence of high concentrations of sorbitol and the natural gum, which however are at the same time essential features of EP2730168 compositions.

Therefore, the object of the present invention is to provide a further improved disinfectant product in powder form, that is effective as, or even more than, the aforementioned composition of the prior art, and which, at the same time, overcomes the limitations of the same.

SUMMARY OF THE INVENTION

The above object has been achieved by a disinfectant composition as set forth by the present claims.

In particular, the present invention concerns a disinfectant composition in powder form comprising a disinfectant agent and excipients comprising sorbitol, a thickener, an amino acid surfactant and an anti-caking agent.

The invention is also directed to a disinfectant solution comprising the disinfectant composition of the invention and an aqueous medium, preferably water.

In another aspect, the present invention concerns a kit comprising a first container containing the disinfectant agent, and a second, separate, container containing the excipients of the invention; moreover, the invention concerns a method of preparing a disinfectant product by using said kit.

In an additional aspect, the present invention concerns the composition, solution or kit as a bactericidal agent in the post-milking teat disinfection treatment of dairy animals. With the term "dairy animals", in the present invention, it is meant to include non-human animals for milk production. Dairy animals are preferably cattle, sheep, goats, buffaloes, camels, yaks, horses, reindeers and donkeys; more preferred dairy animals are cattle, sheep and goats. The characteristics and advantages of the present invention will be evident from the detailed description given below, and from the illustrative non-limiting working examples.

DETAILED DESCRIPTION OF THE INVENTION

The disinfectant composition of the invention is in powder form comprising a disinfectant agent and excipients, said excipients comprising an amino acid surfactant, a thickener, sorbitol, and an anti-caking agent, wherein the amino acid surfactant and the thickener are in a weight ratio of 0.02:1 to 0.6:1, and the amino acid surfactant and sorbitol are in a weight ratio of 0.03:1 to 0.5:1.

Surprisingly, the combination of the above excipients, in the indicated ratios, together with said disinfectant agent, provides a disinfectant composition with high flowability of the powder and long shelf-life; moreover, after dissolution of the composition in an aqueous medium, a highly homogeneous solution can be obtained which also has high in-use stability and has even improved disinfectant activity and emollient effect, compared to the compositions of the prior art.

Advantageously, the composition of the invention is highly stable, homogeneous and flowable (i.e., lumps of powder do not form). Moreover, the composition of the invention effectively adheres to the surfaces to be disinfected, including the teats of dairy animals, so that the contact time is prolonged.

These advantages are achieved because of the combination of active agent and specific excipients, according to the present invention, wherein in particular, the surfactant allows the composition to be spread without forming eyelets and bubbles, that negatively affect the adhesion and cause detachments from the skin or surfaces to be treated.

Preferably, the amino acid surfactant is selected from potassium cocoyl glycinate, sodium cocoyl glycinate, sodium lauroyl sarcosinate, cocoyl alanine TEA salt, sodium cocoyl glutamate, sodium lauroyl glutamate, and mixtures thereof. According to preferred embodiments, said surfactant is sodium cocoyl glutamate.

Preferably, said disinfectant agent is selected from the group consisting of chloramine T, calcium chloride, calcium hypochlorite, sodium dichloro isocyanurate, sodium trichloro isocyanurate, and mixtures thereof. In preferred embodiments, said disinfectant agent is chloramine T.

Preferably, said thickener is selected from the group consisting of Arabic gum, tragacanth, guar gum, xanthan gum, modified starch, modified cellulose, carboxymethylcellulose, propylene glycol alginate, associative polyurethane thickeners, modified fatty acids, inorganic saline thickeners, alkanolamide thickeners, amine oxide thickeners, and mixtures thereof.

According to preferred embodiments, said thickener is selected from the group consisting of Arabic gum, tragacanth, guar gum, xanthan gum, and mixtures thereof. More preferably, said thickener is xanthan gum. In these preferred embodiments, advantageously, the thickener and surfactant are natural compounds having a high skin tolerability.

The composition of the invention preferably comprises disinfectant agent at a concentration of 10-15 wt%, more preferably of 12-14 wt%, based on the weight of the composition. Preferably, sorbitol and disinfectant agent are present in a weight ratio of at least 3:1, preferably of 3.5:1 to 7:1. More preferably, the sorbitol and disinfectant agent are in a weight ratio of 4:1 to 6:1.

The amount of anti-caking agent present in the composition of the invention is suitable to improve flowability of the powder, also in the presence of the hygroscopic sorbitol and thickener. This results in a prolonged shelf-life and long-term storage stability of the composition.

Preferably, the composition comprises the anti-caking agent and sorbitol in a weight ratio of at least 0.01:1, more preferably in a weight ratio of anti-caking agent to sorbitol of up to 0.1:1. Preferably, the composition comprises the anti-caking agent and the thickener in a weight ratio of at least 0.01:1, more preferably in a weight ratio of anti-caking agent to sorbitol of up to 0.2:1.

The anti-caking agent in the composition of the invention is selected from the group consisting of silicon dioxide, tricalcium phosphate, hydrophobic starch derivatives, cellulose powder, calcium silicate, magnesium silicate, aluminium silicate, sodium silicate, sodium bicarbonate, sodium ferrocyanide, potassium ferrocyanide, calcium ferrocyanide, magnesium trisilicate, talc, sodium aluminosilicate, potassium aluminosilicate, calcium aluminosilicate, bentonite, stearic acid, polydimethylsiloxane, polyacrylic acid and respective sodium salts, sodium polyalkyl naphthalene sulphonate, and mixtures thereof. In preferred embodiments, the anti-caking agent is silicon dioxide.

Preferably, the composition of the invention comprises the amino acid surfactant in a weight ratio of 0.05:1 to 0.3:1, to the thickener; and/or in a weight ratio 0.05:1 to 0.2:1, to sorbitol. These weight ratios have resulted to assure the best performances in terms of adhesion to the skin, without forming eyelets and bubbles.

Preferably, the powder composition comprises sorbitol and thickener in a weight ratio of at least 1.4:1, more preferably of 1.5:1 to 2:1, most preferably of 1.6:1 to 1.8:1.

Preferably, the composition of the invention comprises the amino acid surfactant in a weight ratio of 0.05:1 to 1:1, more preferably of 0.1:1 to 0.5:1, to disinfectant agent.

In preferred embodiments, the composition comprises 30-60 wt% sorbitol, 20-40 wt% thickener, 12-14 wt% disinfectant agent, 0.5-10 wt% of amino acid surfactant and 0.5-3% of anti-caking agent, based on the total weight of the composition.

More preferably, the composition comprises 35-55 wt% sorbitol, 25-35 wt% thickener, 12-14 wt% disinfectant agent, 2-6 wt% of amino acid surfactant and 1-2,5% of anti-caking agent, based on the total weight of the composition.

Preferably, the powder composition further comprises a powder colorant. Indeed, advantageously, following the solubilisation of the composition in an aqueous means prior to use, said colorant gives the composition an intense colour thus allowing the treated areas to be traced. More preferably, once in water, said colorant presents an intense blue colour. In the composition of the invention, the colorant can be present in an amount of 0.01-10 wt%, on to the total weight of the composition; preferably the colorant is present in an amount of 1-6 wt%. A higher concentration of colorant guarantees an evident marking of the surfaces treated, such as the teats of the dairy animals.

According to particularly preferred embodiments, the disinfectant composition of the invention comprises:

- 30-60 wt% sorbitol,
- 20-40 wt% thickener,
- 10-15 wt% disinfectant agent,
- 0.5-3 wt% anti-caking agent,
- 0.5-10 wt% surfactant, and
- 0.01-10 wt% colorant,

based on the total weight of the composition.

More preferably, the disinfectant composition of the invention comprises:

- 35-55 wt% sorbitol,
- 25-35 wt% thickener,
- 10-15 wt% disinfectant agent,
- 1.0-2.5 wt% anti-caking agent,
- 2-6 wt% surfactant, and
- 1-6 wt% colorant,

based on the total weight of the composition.

Clearly the above compositions are meant to include the indicated ingredients, each in an amount suitable to not exceed the total weight of 100 wt%.

The invention also concerns a unit dose of the above-described powder composition comprising:

- 10-15 g disinfectant agent,
- 20-40 g thickener,
- 30-60 g sorbitol,
- 0.5-3 g anti-caking agent,
- 0.5-10 g surfactant, and
- 0.01-10 g colorant.

Preferably, said unit dose comprises:

- 10-15 g disinfectant agent,
- 25-35 g thickener,
- 35-55 g sorbitol,
- 1.0-2.5 g anti-caking agent,
- 2-6 g surfactant, and
- 1-6 g colorant.

According to preferred embodiments, said unit dose comprises 100 g of the powder composition of the invention.

In certain embodiments, the composition of the invention, as well as the unit dose, consists essentially of disinfectant agent, thickener, amino acid surfactant, sorbitol, and an anti-caking agent, and optionally a colorant. The expression "consists essentially of' means that these are the only ingredients present in the composition of the invention which play an active role in the disinfection and in assuring the stability and flowability of the powder, and the homogeneity of the product after dissolution of the powder in water, while any other components do not interfere with this action and can be mixed therewith.

In other embodiments, the composition of the invention, as well as the unit dose, consists of disinfectant agent, thickener, amino acid surfactant, sorbitol, an anti-caking agent, and a colorant.

The composition of the invention, employing a natural surfactant, maintains a high skin tolerability.

In a further aspect, the present invention concerns in fact the use of said composition for the post-milking teat disinfection of dairy animals.

In a further aspect, therefore the invention relates to the use of the powder composition of the invention in the veterinary field, since it allowed significantly satisfactory results to be achieved in the post-milking disinfection of dairy animal teats, having shown an optimal bactericidal effect, without causing any skin irritation.

The powder composition of the invention therefore finds advantageous and convenient application as anti-inflammatory and antibacterial agent in the treatment for the prevention of animal conditions such as mastitis.

In such uses, said composition, or said unit dose, are preliminarily dispersed in an aqueous medium, preferably water, to form a solution that jellifies, prior to application.

In particular, the composition, as well as the unit dose, of the present invention is prepared by mixing the powder ingredients in the weight ratio as defined above.

An amount of 100g of the powder composition is suitable for being dissolved in a 3-7 litres volume of an aqueous medium, preferably about 5 litres, wherein said aqueous medium is preferably water.

Therefore, preferably, said composition, or said unit dose, is dispersed in an aqueous medium at a concentration of 10-30 g/l, more preferably 15-25 g/l; after about at least 3 hours, preferably after at least 6 hours of rest, the mixture forms a gel that can be applied to the surfaces to be disinfected, e.g., to teats of the animals to treat after milking, for example by dipping.

It is noted that the active ingredient is homogeneously dispersed in the gel that forms after the addition of the composition of the invention to an aqueous solution. Surprisingly, it was found that even greater homogeneity can be achieved by first dissolving the active agent in the aqueous solution, and then adding the excipients.

The present invention is therefore directed also to a disinfecting kit comprising a first container containing the disinfectant agent, and a second, separate, container containing the excipients of the invention.

Moreover, the invention concerns a method of preparing a disinfectant product by using said kit, comprising the following phases:

- providing the kit of the invention,
- dissolving the disinfectant agent in an aqueous solution, preferably in water, inside a container, such as a tank;
- adding the excipients to the aqueous solution of disinfectant agent and vigorously shaking until complete dispersion;
- letting the obtained solution to form a gel, prior to application, preferably by keeping it at room temperature for at least 3 hours, more preferably at least 6 hours, most

preferably for 6-8 hours.

Before the use, the disinfectant solution is preferably poured into a dipper for applying the product to the milk cow's teats.

The composition and the ready-to-use solution of the invention have high stability, therefore both the composition and the ready-to-use reconstituted solution can be stored for long before and during use. This allows the use of the entire solution and the consequent reduction in product waste (and consequent reduction in chemical waste).

It is to be understood that all aspects identified as preferred and advantageous for the composition of the invention, are to be deemed analogously preferred and advantageous also for the unit dose, and both the medical and non-medical uses of said composition. Working examples of the present invention provided for illustrative purposes are reported herein below.

## EXAMPLES

### Example 1

100 g of a composition for veterinary use in powder form were prepared in accordance with the present invention, by mixing:

- 13.5 g of chloramine-T,
- 31.5 g of xanthan gum,
- 47.7 g of sorbitol,
- 2.5 g of sodium cocoyl glutamate,
- 1.8 g of silicon dioxide, and
- 3 g of colorant.

### Example 2

A kit according to the invention was prepared. In particular, a powder excipients' composition in powder form was prepared by mixing:

- 28.3 g of xanthan gum,
- 49.2 g of sorbitol,
- 4 g of sodium cocoyl glutamate,
- 1.5 g of silicon dioxide, and
- 4 g of colorant.

Said powder excipients' composition was then transferred into a first container, specifically an envelope.

At the same time, 12 g of chloramine-T was transferred into a second container, specifically an envelope.

The resulting kit ready-to-use then included said first and second envelopes.

### Example 3

100 g of a composition for veterinary use in powder form were prepared in accordance with the present invention, by mixing:

- 50 g of sorbitol,
- 28 g of guar gum,
- 3 g of sodium lauroyl glutamate,
- 13.5 g of sodium dichloro isocyanurate,
- 2.0 g of calcium silicate, and
- 3.5 g of colorant.

### Example 4

The composition of Example 1 was dispersed in 5 litres of water and allowed to stand for 4 hours.

The resulting gel mixture was then used in the post-milking disinfection treatment of the teats of 100 goats. No redness or irritation following application was observed, demonstrating that the composition of the invention is perfectly tolerated by the skin.

In addition, the goats periodically treated with the same composition did not develop either local ulceration or mastitis over time, thus demonstrating that the composition of the invention also allows an effective prevention action against these conditions.

### Example 5

13 g of chloramine-T were dispersed in 5 litres of water, then the excipients of the composition of Example 2 was added. The solution obtained was shaken vigorously and allowed to stand for 6 hours.

The resulting gel mixture was then used in the post-milking disinfection treatment of the teats of 100 goats. No redness or irritation following application was observed, demonstrating that the composition of the invention is perfectly tolerated by the skin.

In addition, the goats periodically treated with the same composition did not develop either local ulceration or mastitis over time, thus demonstrating that the composition of the invention also allows an effective prevention action against these conditions.

### Example 6

The composition of Example 3 was dispersed in 5 litres of water and allowed to stand for 6 hours.

The resulting gel mixture was then used in the post-milking disinfection treatment of the teats of 100 goats. No redness or irritation following application was observed, demonstrating that the composition of the invention is perfectly tolerated by the skin.

In addition, the goats periodically treated with the same composition did not develop either local ulceration or mastitis over time, thus demonstrating that the composition of the invention also allows an effective prevention action against these conditions.

**Example 7**

**In-use stability test**

Integrity of products in multidose containers after the first opening/broaching is an important quality issue. In-use stability testing was then carried out to establish the period of time during which the product of the invention may be used whilst retaining quality within an acceptable specification once the container is opened or broached (in-use shelf life).

A first container containing 13 g of chloramine-T was opened and its content was dispersed in 5 liters of water, then a second container containing the excipients of the composition of Example 2 was opened and its content was added to the solution of chloramine-T in water. The solution obtained was shaken vigorously and allowed to stand for 4 hours. The resulting gel mixture was then used as test item for the in-use stability test.

The gel was kept at room temperature (20°C) up to 12 days; at the end of the test total viable count was determined, while Chloramine-T assay was carried out at several time points during the 12 days of testing, in particular at 6, 24, 72, 168, 240 hours after the dissolution of the powder and last at the end of the 12 days (288 hours time-point), as indicated in Table 1 that follows.

Also, viscosity was determined at the beginning and at the end of the test (T6 and T288), as well as visual evaluation of the appearance of the test item solution.

**Table 1.**

| Condition | Time point (hours) | | | | | |
|---|---|---|---|---|---|---|
| | T6 | T24 | T72 | T168 | T240 | T288 |
| Room Temperature | X, Y | X | X | X | X | X, Y, Z |
| | | | | | | |
| X | Chloramine-T assay determination | | | | | |
| Y | Viscosity at 20°C determination | | | | | |
| Z | Total viable count (TVC) | | | | | |
| X,Y and Z = sampling and analysis | | | | | | |

Detailed description of the assays and results are provided hereafter.

Viscosity determination:

About 200 ml of test item solution were withdrawn from the tank and thermostated at 20°C A rotational viscometer was employed (Viscometer Fungilab VISCO BASIC Plus R), with R2 spindle and speed set at 20 rpm 8value kept after 50s).

Chloramine-T assay determination

The assay was performed according to the titrimetric method (redox titration)

RESULTS

The test item powder appears as a white-grey fine powder without aggregates.

The results in table 2 refer to the gel mixture stored at room temperature for 288 hours.

**Table 2**

| TESTS | METHOD | T6 h | T24 h | T72 h | T168 h | T240 h | T288 h |
|---|---|---|---|---|---|---|---|
| SAMPLING DATE | | 19/06/15 | 20/06/15 | 22/06/15 | 26/06/15 | 29/06/15 | 01/07/15 |
| Viscosity (cP) | Rotational | 955.7 | | | | | 1341.1 |
| Total viable count (cfu/ml) | EP 8 (2.6.12) | | | | | | <10 |
| Active ingredient assay % (w/w) — *Chloramine T anhydrous* | 2012/492 AMi-MdP rev1 | 0.15 | 0.17 | 0.17 | 0.16 | 0.16 | 0.15 |
| Active ingredient assay % (w/w) — *Chloramine T trihydrate* | 2012/492 AMi-MdP rev1 | 0.18 | 0.21 | 0.21 | 0.20 | 0.19 | 0.19 |
| expressed as: *Active Chlorine* | 2012/492 AMi-MdP rev1 | 0.046 | 0.053 | 0.053 | 0.049 | 0.049 | 0.047 |

The stability study shows that after 6 hours the assay of the active is still low. It increases after 24 hours and slowly decreases in 12 days of conservation. The viscosity increases with time and the total viable count is compliant with the EP 2.6.12 specifications.

**Example 8**

**Accelerated stability test**

Accelerated stability test has been carried what to assess the quality of a test item prepared as the one in Example 7 during time, under the influence of temperature and humidity. The test was conducted at 30°C, at 65% of relative humidity (RH), for 18 weeks. Deviation from temperature set point did not exceed 2°C, and deviation from RH set point did not exceed 5%,

The test item's stability was evaluated at time 0 and after 18 weeks, assessing: the appearance of each container (the one containing chloramine T and the one containing the excipients), appearance of the products therein, the weight loss of the products and the content of chloramine T.

Appearance of the test item and of the packaging was assessed under the lab light. Weight loss was assessed weighing each sample at T0 ($W_{T0}$) and at the final time point ($W_{Tx}$) and compared with their initial value (%) according to the following formula:

$$\text{Weight loss from initial \%} = [(W_{T0} - W_{Tx})*100]/(W_{T0})$$

The content of chloramine T was assessed as described in Example 7.

RESULTS

Chloramine T consists of a white crystalline powder contained in a white square sealed bad. After 18 weeks at 30°C/65%RH the sample did not present variations from T0.

Also the packaging showed no leakage, no ballooning, no panelling and no deformations. The excipients composition consists of a beige granular powder contained in a white rectangular sealed bad.

After 18 weeks at 30°C/65%RH the sample did not present variations from T0.

Also the packaging showed no leakage, no ballooning, no panelling and no deformations. The content of active ingredient was described in Table 3.:

**Table 3.**

| | Available Chlorine (%w/w) | | | CHLORAMINE T tri-hydrated (%w/w) | | | CHLORAMINE T anhydrous (%w/w) | | |
|---|---|---|---|---|---|---|---|---|---|
| | T0 | T8w | T18w | T0 | T8w | T18w | T0 | T8w | T18w |
| | 24.57 | - | 24.59 | 78.88 | - | 97.68 | 97.61 | - | 78.94 |
| | 24.49 | 0.18 | 24.66 | 78.64 | 0.59 | 97.99 | 97.31 | 0.73 | 79.18 |
| average | **24.53** | **0.18** | **24.62** | **78.76** | **0.59** | **97.83** | **97.46** | **0.73** | **79.06** |
| $|X_1-X_2|$ | 0.07 | - | 0,08 | 0.24 | - | 0.30 | 0.29 | - | 0.24 |
| | Tx/T0 % | - | 100.4% | - | | 100.4% | - | | 100.4% |

In summary, after 18 weeks no significant variations in the appearance and weight were observed for both envelopes containing the active substance and excipients. The content of active chloramine T was 100.4% compared to T0.

**Claims**

1. A disinfectant composition in powder form comprising a disinfectant agent and excipients, said excipients comprising an amino acid surfactant, a thickener, sorbitol, and an anti-caking agent, wherein the amino acid surfactant and the thickener are in a weight ratio of 0.02:1 to 0.6:1, and the amino acid surfactant and sorbitol are in a weight ratio of 0.03:1 to 0.5:1.

2. The disinfectant composition of claim 1, wherein the amino acid surfactant is selected from the group consisting of potassium cocoyl glycinate, sodium cocoyl glycinate, sodium lauroyl sarcosinate, cocoyl alanine TEA salt, sodium cocoyl glutamate, sodium lauroyl glutamate, and mixtures thereof, preferably wherein the amino acid surfactant is sodium cocoyl glutamate.

3. The disinfectant composition of claim 1 or 2, wherein the surfactant and the thickener are in a weight ratio of 0.05:1 to 0.3:1, and wherein the surfactant and sorbitol are in a weight ratio of 0.05:1 to 0.2:1.

4. The disinfectant composition of any one of claims 1-3, comprising 30-60 wt% sorbitol, 20-40 wt% thickener, 12-14 wt% disinfectant agent, 0.5-10 wt% of amino acid surfactant and 0.5-3% of anti-caking agent, based on the total weight of the composition.

5. The disinfectant composition of any one of claims 1-4, wherein said thickener is selected from the group consisting of Arabic gum, tragacanth, guar gum, xanthan gum, modified starch, modified cellulose, carboxymethylcellulose, propylene glycol alginate, associative polyurethane thickeners, modified fatty acids, inorganic saline thickeners, alkanolamide thickeners, amine oxide thickeners, and mixtures thereof, preferably is selected from the group consisting of Arabic gum, tragacanth, guar gum, xanthan gum, and mixtures thereof.

6. The disinfectant composition of any one of claims 1-5, wherein said disinfectant agent is selected from the group consisting of chloramine T, calcium chloride, calcium hypochlorite, sodium dichloro isocyanurate, sodium trichloro isocyanurate, and mixtures thereof, preferably is chloramine T.

7. The disinfectant composition of any one of claims 1-6, wherein the anti-caking agent and sorbitol are in a weight ratio of at least 0.01:1, preferably of 0.01:1 to 0.1:1.

8. The disinfectant composition of any one of claims 1-7, wherein sorbitol and disinfectant agent are in a weight ratio of at least 3:1, preferably of 3.5:1 to 7:1.

9. The disinfectant composition of any one of claims 1-8, wherein the surfactant and disinfectant agent are in a weight ratio of 0.05:1 to 1:1, preferably of 0.1:1 to 0.5:1.

10. The disinfectant composition of any one of claims 1-9, comprising 12-14 wt% disinfectant agent, 25-35 wt% of thickener, 2-6 wt% of amino acid surfactant, 35-55 wt% sorbitol, and 1-2,5% of anti-caking agent, based on the total weight of the composition.

11. The disinfectant composition of any one of claims 1-10, further comprising a powder colorant.

12. The disinfectant composition of any one of claims 1-11, in the form of a unit dose, comprising:

  - 10-15 g disinfectant agent,
  - 25-35 g thickener,
  - 35-55 g sorbitol,
  - 1.0-2.5 g anti-caking agent,
  - 2-6 g surfactant, and
  - 1-6 g colorant.

13. A disinfectant kit comprising a first container containing the disinfectant agent, and a second container containing the excipients of the composition of any one of claims 1-12, the disinfectant kit, optionally comprising water.

14. A disinfectant solution comprising the disinfectant composition of any one of claims 1-12 and an aqueous medium, preferably water, wherein the disinfectant composition is dissolved in said aqueous medium at a concentration of 10-30 g/l, preferably of 15-25 g/l.

15. The composition of any one of claims 1-12 or the kit of claim 13 or the solution of claim 14, for use as a bactericidal agent in the post-milking teat disinfection treatment of dairy animals.

Europäisches
Patentamt

European
Patent Office

Office européen
des brevets

# EUROPEAN SEARCH REPORT

Application Number

EP 22 20 7309

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | EP 2 730 168 A1 (ICF SRL [IT])<br>14 May 2014 (2014-05-14)<br>* abstract *<br>* paragraphs [0001], [0009] – [0032];<br>claims 1, 9, 11-14; examples 3-4 * | 1-15 | INV.<br>A01N59/00<br>A01N41/06<br>A01N25/12<br>A01N25/24<br>A01N25/30<br>A01P1/00 |
| Y | EP 1 932 425 A2 (ICF SRL [IT])<br>18 June 2008 (2008-06-18)<br>* abstract *<br>* paragraphs [0001], [0005] – [0021] * | 1-15 | |
| Y | US 2004/225017 A1 (SCHNEIDER CHARLES A<br>[US]) 11 November 2004 (2004-11-11)<br>* abstract *<br>* paragraphs [0002], [0010] – [0018],<br>[0044] – [0045] *<br>* paragraphs [0053] – [0055]; claims<br>18-20; examples 1-2 * | 1-15 | |
| Y | US 4 446 153 A (YANG KIM W [US])<br>1 May 1984 (1984-05-01)<br>* abstract *<br>* column 2, lines 13-39 *<br>* column 3, lines 27-52 *<br>* column 6, lines 58-68; table 3 *<br>* claims 1, 5, 7-8, 12 * | 1-15 | TECHNICAL FIELDS SEARCHED (IPC)<br><br>A01N<br>A01P |
| Y | WO 2016/124764 A1 (HYGIENIX BV)<br>11 August 2016 (2016-08-11)<br>* abstract *<br>* page 1, lines 6-12 *<br>* page 2, line 34 – page 3, line 29 *<br>* page 9, lines 14-30 *<br>* page 16, lines 9-19 *<br>* examples 1-5 * | 1-15 | |

The present search report has been drawn up for all claims

| Place of search | Date of completion of the search | Examiner |
|---|---|---|
| Munich | 28 April 2023 | Hateley, Martin |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 22 20 7309

28-04-2023

| Patent document cited in search report | | | Publication date | Patent family member(s) | | | Publication date |
|---|---|---|---|---|---|---|---|
| EP 2730168 | A1 | | 14-05-2014 | AU | 2013343676 | A1 | 14-05-2015 |
| | | | | BR | 112015009310 | A2 | 04-07-2017 |
| | | | | CA | 2888887 | A1 | 15-05-2014 |
| | | | | EA | 201590596 | A1 | 30-10-2015 |
| | | | | EP | 2730168 | A1 | 14-05-2014 |
| | | | | ES | 2619428 | T3 | 26-06-2017 |
| | | | | HR | P20170409 | T1 | 02-06-2017 |
| | | | | HU | E033136 | T2 | 28-11-2017 |
| | | | | JP | 2015535298 | A | 10-12-2015 |
| | | | | PL | 2730168 | T3 | 31-07-2017 |
| | | | | PT | 2730168 | T | 17-03-2017 |
| | | | | US | 2015297542 | A1 | 22-10-2015 |
| | | | | WO | 2014072315 | A1 | 15-05-2014 |
| EP 1932425 | A2 | | 18-06-2008 | NONE | | | |
| US 2004225017 | A1 | | 11-11-2004 | NONE | | | |
| US 4446153 | A | | 01-05-1984 | CA | 1219805 | A | 31-03-1987 |
| | | | | US | 4446153 | A | 01-05-1984 |
| WO 2016124764 | A1 | | 11-08-2016 | BR | 112017016729 | A2 | 10-04-2018 |
| | | | | CA | 2974892 | A1 | 11-08-2016 |
| | | | | CN | 107454822 | A | 08-12-2017 |
| | | | | DK | 3253207 | T3 | 24-02-2020 |
| | | | | EP | 3253207 | A1 | 13-12-2017 |
| | | | | ES | 2772931 | T3 | 08-07-2020 |
| | | | | KR | 20170136503 | A | 11-12-2017 |
| | | | | PL | 3253207 | T3 | 18-05-2020 |
| | | | | RU | 2712186 | C1 | 24-01-2020 |
| | | | | US | 2018027812 | A1 | 01-02-2018 |
| | | | | WO | 2016124764 | A1 | 11-08-2016 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2730168 A